# EUROPEAN PATENT APPLICATION

(11) **EP 3 696 546 A1**
(43) Date of publication of application: **19.08.2020**
(21) Application number: 18865571.6
(22) Date of filing: 15.10.2018
(51) Int. Cl.: G01N 33/569, G01N 33/68, C12Q 1/6881

(54) **METHOD FOR SELECTING HIGHLY EFFICIENT STEM CELL, USING PROTEIN MARKER GRP78**

(30) Priority: 13.10.2017 KR 20170133613
(71) Applicant: Nibec Co., Ltd., Chungcheongbuk-do 27816 (KR); Chungbuk National University Industry-Academic Cooperation Foundation, Cheongju-si, Chungcheongbuk-do 28644 (KR); Seoul National University R & DB Foundation, Seoul 08826 (KR)
(72) Inventor: PARK, Yoon Shin, Cheongju-si Chungcheongbuk-do 28415 (KR); PARK, Yoon Jeong, Seoul 08291 (KR); CHUNG, Chong-Pyoung, Seoul 05618 (KR); LEE, Jue-Yeon, Gwacheon-si Gyeonggi-do 13831 (KR); CHOI, Da Hyeon, Cheongju-si Chungcheongbuk-do 28645 (KR)
(74) Representative: Latscha Schöllhorn Partner AG
(86) International application number: PCT/KR2018/012117
(87) International publication number: WO 2019/074342

(57) **Abstract**

The present invention relates to a method of screening highly efficiently stem cells using the protein marker GRP78, and more particularly, to a method of removing old stem cells with decreased expression of GRP78 and isolating only highly efficient stem cells. The use of the protein marker GRP78 according to the present invention makes it possible to isolate only highly efficient stem cells by removing old stem cells. Thus, the protein marker GRP78 may be used as an effective marker for controlling the quality of stem cell therapy products and evaluating the stability and effectiveness thereof, and is useful in the production of stem cell therapy products having excellent therapeutic efficacy.

## Description

### Technical Field

The present invention relates to a method of screening highly efficiently stem cells using the protein marker GRP78, and more particularly, to a method of removing old stem cells with decreased expression of GRP78 and isolating only highly efficient stem cells.

### Background Art

In recent years, various studies have been conducted on the possibility of using stem cells as cell therapy by differentiating stem cells, such as embryonic stem cells and adult stem cells, into various cells. Multipotent embryonic stem cells have attracted attention as cell therapy products due to their ability to differentiate into various cells. However, the use of embryonic stem cells poses ethical problems, and hence it is difficult to use these embryonic stem cells as cell therapy in practice. To avoid such ethical problems, studies on the use of adult stem cells have been actively conducted (Trends in Neurosciences 23:450, 2000).

Although adult stem cells have disadvantages in that when these cells are transplanted into other persons, they carry the risk of infection and have a relatively low differentiation potential, these cells have advantages in that they can be obtained in large numbers and are very safe for medical applications. Specifically, even when these cells are transplanted into the body for organ regeneration, they do not cause cancer, and do not cause immune rejection because they have originated from the adult body. Thus, these adult stem cells can be used for autologous transplantation. In addition, adult stem cells have site-specific differentiation potential according to the characteristics of surrounding tissue, and do not cause cancer even when they are injected in an undifferentiated state. Thus, these adult stem cells advantageously have the potential to produce necessary cells immediately after transplantation and also have self-renewal potential to create and store undifferentiated stem cells, if necessary (JACC: Basic to Translational Science 2:702-716, 2017).

During development of stem cells into a cell therapy product, a process of culturing isolated stem cells for a long period of time is essential in order to obtain the number of cells required to have an optimal therapeutic effect. Due to the long-term culture, development of the cell therapy product is inevitably performed in a state in which a large number of old cells is mixed or cells at various passages are mixed. This can cause problems in the stability and effectiveness of the stem cell therapy product, as well as quality control (European Cells and Materials 31:136-159, 2016). Therefore, in order to develop a cell therapy product using adult stem cells, a process of screening old cells mixed due to long-term culture is necessary. Although various efforts have, in fact, been made to control the quality of stem cell therapy products, established clear markers are still insufficient.

Accordingly, the present inventors have made extensive efforts to find markers, which may be used to remove old stem cells and screen only highly efficient stem cells, and as a result, have found that the protein GRP78 associated with glucose metabolism can be used as a senescence-related stem cell marker during long-term culture of adult stem cells as the number of passages increases, thereby completing the present invention.

The above information disclosed in this Background section is only for enhancement of understanding of the background of the present invention. Therefore, it may not contain information that forms a conventional art that is already known in the art to which the present invention pertains.

### Summary of the Invention

An object of the present invention is to provide the marker GRP78 for developing a stem cell therapy product using adult stem cells and controlling the quality of the stem cell therapy product.

Another object of the present invention is to provide a method of removing old stem cells and isolating highly efficient stem cells to produce a stem cell therapy product having excellent efficacy.

Still another object of the present invention is to provide a composition and a kit for screening highly efficient stem cells using the protein marker GRP78.

To achieve the above objects, the present invention provides a method of isolating highly efficient stem cells using an antibody or aptamer specific for GRP78.

The present invention also provides a composition for screening highly efficient stem cells, the composition comprising an agent capable of measuring the expression level of mRNA of GRP78 gene or a protein encoded by the gene.

The present invention also provides a kit for screening highly efficient stem cells, the kit comprising the composition.

The present invention also provides a method for screening highly efficient stem cells, the method comprising steps of: (a) extracting DNA or protein from stem cells; (b) measuring the expression level of mRNA of GRP78 gene or a protein encoded by the gene; (c) comparing the measured expression level of mRNA of the gene or the protein encoded by the gene with a control; (d) selecting the stem cells as highly efficient stem cells if the expression level increases compared to the control.

### Brief Description of Drawings

FIG. 1a shows the change in morphology of adult stem cells by senescence.
FIG. 1b shows changes in adult stem cell-specific cell markers of young cells and old cells.
FIG. 1c shows the results of examining whether adult stem cells have the characteristics of old cells during passage.
FIG. 2 shows GRP78 significantly obtained from the results of proteomic analysis of adult stem cells at various passages.
FIG. 3 shows changes in the GRP78 gene level in old adult stem cells.
FIG. 4 shows changes in the GRP78 protein level in old adult stem cells.
FIG. 5 shows the expression locations of GRP78 in cells as senescence progresses.
FIG. 6 shows that the GRP78 protein level is a change specific to adult stem cells.

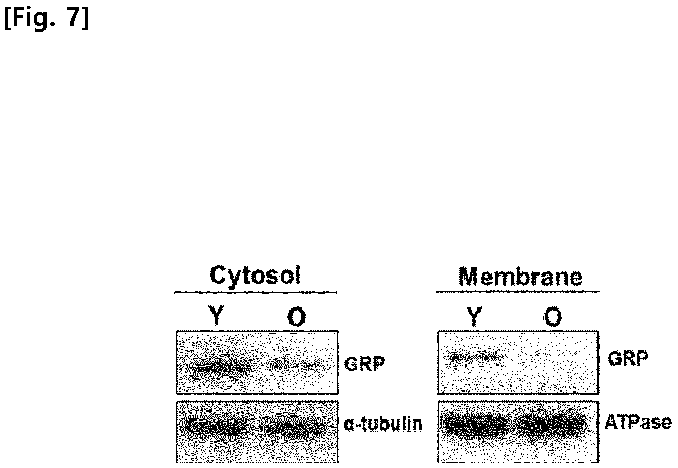

FIG. 7 shows the expression level of GRP78 protein in each cell region.

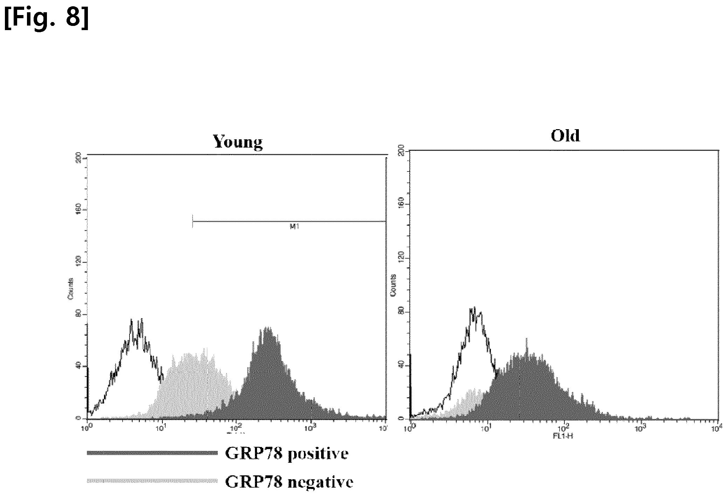

FIG. 8 shows a GRP78 surface marker after isolating GRP78 from adult stem cells by an isolation method using a magnet.

### Detailed Description and Preferred Embodiments of the Invention

Unless otherwise defined, all the technical and scientific terms used in the present specification have the same meanings as commonly understood by those skilled in the art to which the present disclosure pertains. In general, the nomenclature used in the present specification is well known and commonly used in the art.

In the present invention, GRP78 (glucose-regulated protein 78) showing a significant expression change in old stem cells was identified by examining proteomic changes as senescence of adult stem cells progressed. It was confirmed that the expressions of GRP78 gene and protein in old stem cells significantly decreased, and in old stem cells, the translocation of GRP78 protein to the cytoplasm and membrane decreased, and the expression of GRP78 protein in the cytoplasm and membrane significantly decreased as senescence progressed. In addition, it could be confirmed that the use of MACS (magnetic-activated cell sorting) could remove old stem cells expressing no GRP78 protein and selectively isolate only highly efficient stem cells expressing the GRP78 protein in the membrane.

Therefore, one aspect of the present invention is directed to a method of isolating highly efficient stem cells using an antibody or aptamer specific to GRP78.

In the present invention, the method may comprise steps of: (a) binding a GRP78-specific primary antibody to stem cells; (b) binding a secondary antibody-immobilized bead to the primary antibody; and (c) isolating GRP78-expressing stem cells using MACS (magnetic-activated cell sorting).

In the present invention, the highly efficient stem cells may express GRP78 in the membrane.

In the present invention, step (c) may further comprise a step of removing stem cells expressing a low level of GRP78 in the cell membrane, and the stem cells expressing a low level of GRP78 in the cell membrane may be old stem cells.

The nucleotide sequence and amino acid sequence of GRP78 of the present invention may be obtained from a known database such as NCBI's GenBank (e.g., GenBank Accession AAA52614.1).

In the present invention, the stem cells are preferably adult stem cells, but are not limited thereto.

As used herein, the term "stem cells" refers to pluripotent cells capable of differentiating into endodermal, mesodermal and ectodermal cells in animals, or multipotent cells capable of differentiating into closely related cells in tissues or functions. The term refers to a cell capable of dividing into two or more new cells while having self-renewal ability, and the stem cells may be classified into totipotent stem cells, pluripotent stem cells, and multipotent stem cells.

As used herein, the term "adult stem cells" refers to multipotent cells obtained by isolating stem cells from each tissue and culturing the isolated stem cells *in vitro,* and includes bone marrow stem cells, retinal stem cells, retinal Muller glial cells, neural stem cells, and the like. The stem cells may be derived from mammals, including humans and primates, as well as livestock such as cattle, pigs, sheep, horses, dogs, mouse, rats, and cats. Preferably, the stem cells may be derived from humans.

As used herein, the term "senescence" means that differentiation or proliferation capacity of adult stem cells decreases due to an increase in the age of an individual or an increase in the number of passages.

As used herein, the term "passage" means replacing a culture vessel or dividing and culturing a cell population, in a method in which a portion of cells is periodically transferred to a fresh culture vessel in order to continuously culture the cells in a healthy state for a long period of time, and then the cells are continuously subcultured while the culture medium is replaced. One passage refers to replacing the culture vessel once or dividing and culturing the cell population. In the present invention, low-passage or young-passage stem cells may refer to stem cells having a high differentiation ability or therapeutic effect, and high-passage or old-passage stem cells may refer to stem cells having a low differentiation ability or therapeutic effect, but are not limited thereto.

In the present invention, the young passage may have a passage number of 0 to 3, preferably 0 to 2. In addition, the old passage may have a passage number of 15 to 25, preferably 20 to 25.

That is, "old stem cells" in the present invention refers to stem cells in which the differentiation or proliferation ability of adult stem cells has decreased due to an increase in the number of passages, and "highly efficient stem cells" refers to stem cells that have excellent efficacy as cell therapy products because the adult stem cells therein have a very high differentiation or proliferation ability. In other words, "highly efficient stem cells" refers to those that exhibit differentiation or proliferation capacity equivalent to young-passage stem cells.

Another aspect of the present invention is directed to a composition for screening highly efficient stem cells, the composition comprising an agent capable of measuring the expression level of mRNA of GRP78 gene or a protein encoded by the gene.

In the present invention, the agent capable of measuring the expression level of mRNA is preferably a primer capable of amplifying the GRP78 gene or a probe that binds specifically to the GRP78 gene, but is not limited thereto.

In addition, the composition may further comprise an amplification means capable of amplifying the DNA of the sample, and may also optionally comprise a means for extracting the gene from the sample. The method of amplifying the DNA of the sample by PCR and the method of extracting the gene from the sample are known in the art, and detailed description thereof is omitted herein.

As used herein, the term "primer" refers to a nucleic acid sequence having a short free 3'-end hydroxyl group, which is a short nucleic acid sequence that may form a base pair with a complementary template and act as a starting point for template strand replication. The primer may initiate DNA synthesis in the presence of a reagent (e.g., DNA polymerase or reverse transcriptase) for polymerization and four different nucleoside triphosphates in a suitable buffer at a suitable temperature. PCR conditions and the lengths of the sense and antisense primers may be modified based on those known in the art.

As used herein, the term "probe" refers to a fragment of nucleic acid such as RNA or DNA, which can bind specifically to mRNA and is several nucleotides to several hundred nucleotides in length. The probe can determine the presence or absence of a specific mRNA, because it is labeled.

The probe can be constructed as an oligonucleotide probe, a single-stranded DNA probe, a double-stranded DNA probe, an RNA probe or the like. A suitable probe and hybridization conditions may be modified based on those known in the art.

The primer or probe of the present invention may be chemically synthesized by a phosphoramidate solid support method or other well-known methods. This nucleic acid sequence may also be modified using many means known in the art. Non-limiting examples of this modification include methylation, capping, substitution with one or more homologues of natural nucleotides, and modification between nucleotides, for example, modification into an uncharged linker (e.g., methyl phosphonate, phosphotriester, phosphoroamidate, carbamate, etc.) or charged linker (e.g., phosphorothioate, phosphorodithioate, etc.).

In the present invention, the agent for measuring the expression level of the protein is preferably an antibody or aptamer specific to the protein encoded by the GRP78 gene, but is not limited thereto.

Examples of a method for qualitative or quantitative detection/measurement at the protein level, which may be used in the present invention, include Western blotting, ELISA, radioimmunoassay, immunodiffusion, immunoelectrophoresis, immunohistostaining, immunoprecipitation assay, complement fixation assay, binding to a labeled antibody in solution/suspension, mass spectrometry, or a protein array using an antibody, etc.

The reagent or agent that is used in this method is known. For example, it may be an antibody, substrate, nucleic acid or peptide aptamer that binds specifically to the marker, or a receptor, ligand or cofactor that interacts specifically with the marker, or an antibody, antibody fragment, aptamer, avidity multimer or peptidomimetics that can bind specifically to the protein.

As used herein, the term "antibody" refers to a protein molecule that may bind specifically to the antigenic site of a protein or peptide molecule. This antibody may be produced by cloning each gene into an expression vector according to a conventional method to obtain a protein encoded by the marker gene and producing the antibody from the obtained protein according to a conventional method. The form of the antibody is not specifically limited, and polyclonal antibodies, monoclonal antibodies, or portions thereof which have antigen binding ability are included in the antibodies of the present invention, and all immunoglobulin antibodies, a special antibody such as humanized antibody, etc. may also be included in the antibodies of the present invention. Furthermore, the antibody includes not only a complete antibody having two full-length light chains and two full-length heavy chains, but also a functional fragment of the antibody molecule. The expression "functional fragment of the antibody molecule" refers to a fragment having at least antigen binding ability, and examples of the functional fragment include Fab, F(ab'), F(ab')2, Fv, etc.

In the present invention, the antibody may be an antibody that can bind specifically to the GRP78 protein. Preferably, it may be a polyclonal antibody, a monoclonal antibody or a portion thereof that can bind specifically to the GRP78 protein.

In the present invention, the highly efficient stem cells may be non-senescent young cells.

In the present invention, the young stem cells may have a passage number of 0 to 3, preferably 0 to 2. In addition, the old stem cells may have a passage number of 15 to 25, preferably 20 to 25.

That is, "old stem cells" in the present invention refers to stem cells in which the differentiation or proliferation ability of adult stem cells has decreased due to an increase in the number of passages, and "highly efficient stem cells" refers to stem cells that have excellent efficacy as cell therapy products because the adult stem cells therein have a very high differentiation or proliferation ability. In other words, "highly efficient stem cells" refers to those that exhibit differentiation or proliferation capacity equivalent to young-passage stem cells.

Still another aspect of the present invention is directed to a kit for screening highly efficient stem cells, the kit comprising an agent capable of measuring the expression level of mRNA of GRP78 gene or a protein encoded by the gene.

In the present invention, the kit may be an RT-PCR kit, a DNA chip kit or a protein chip kit.

The kit of the present invention may be a diagnostic kit composed of one or more compositions, solutions or instruments, which are suitable for analysis methods, and may be an RT-PCR kit, a DNA chip kit or a protein chip kit. The RT-PCR kit may comprise, in addition to each primer pair specific for the marker gene, a test tube or another suitable container, a reaction buffer, deoxynucleotides (dNTPs), enzymes such as Taq-polymerase and reverse transcriptase, DNase or RNase inhibitor, DEPC-water, sterile water, etc. Also, it may comprise a primer pair specific for a gene which is used as a quantitative control. The DNA chip kit may comprise a substrate in which cDNA corresponding to the gene or a fragment thereof is attached as a probe. The substrate may contain cDNA corresponding to a quantification control gene or a fragment thereof.

In addition, the kit according to the present invention may be a diagnostic kit comprising an agent for measuring the protein level, in which the agent for measuring the protein level is preferably an antibody specific to the protein. Thus, the kit comprising the agent for measuring the protein level may be for example, a kit for detection of markers, which comprises essential elements required for carrying out ELISA. This kit may also comprise reagents that may detect antibodies forming "antigen-antibody complexes", for example, labeled secondary antibodies, chromophores, enzymes (e.g., conjugated with antibodies) and their substrates. Also, it may comprise an antibody specific to a control protein for quantification.

Furthermore, the amount of antigen-antibody complexes formed may be quantitatively determined by measuring the signal intensity of a detection label. Such a detection label may be selected from the group consisting of, but not necessarily limited to, enzymes, fluorescent substances, ligands, luminescent substances, microparticles, redox molecules and radioactive isotopes.

Analysis methods for measuring protein levels include, but are not necessarily limited to, Western blotting, ELISA, radioimmunoassay, radioimmunodiffusion, Ouchterlony immunodiffusion assay, rocket immunoelectrophoresis assay, immunohistostaining assay, immunoprecipitation assay, complement fixation assay, fluorescence activated cell sorter (FACS), protein chip assay, etc., and the protein level may be measured using methods known to those skilled in the art.

Yet another aspect of the present invention is directed to a method for screening highly efficient stem cells, the method comprising steps of: (a) extracting a DNA or protein from stem cells; (b) measuring the expression level of mRNA of GRP78 gene or a protein encoded by the gene; (c) comparing the measured expression level of mRNA of the gene or the protein encoded by the gene with a control; and (d) selecting the stem cells as highly efficient stem cells if the expression level increases compared to the control.

In the present invention the mRNA expression of the gene is preferably measured by any one selected from the group consisting of, but not limited to, polymerase chain reaction (PCR), reverse transcription-polymerase chain reaction (RT-PCR), real-time PCR, RNase protection assay (RPA), microarray, and Northern blotting.

In the present invention, the expression level of the protein is preferably measured by any one selected from the group consisting of, but not limited to, Western blotting, radioimmunoassay (RIA), radioimmunodiffusion, enzyme-linked immunosorbent assay (ELISA), immunoprecipitation, flow cytometry, immunofluorescence assay, Ouchterlony assay, complement fixation assay, and protein chip assay.

In the present invention, the control may be old stem cells, and the highly efficient stem cells may be non-senescent young stem cells.

In the present invention, the young stem cells may have a passage number of 0 to 3, preferably 0 to 2. In addition, the old stem cells may have a passage number of 15 to 25, preferably 20 to 25.

That is, "old stem cells" in the present invention refers to stem cells in which the differentiation or proliferation ability of adult stem cells has decreased due to an increase in the number of passages, and "highly efficient stem cells" refers to stem cells that have excellent efficacy as cell therapy products because the adult stem cells therein have a very high differentiation or proliferation ability. In other words, "highly efficient stem cells" refers to those that exhibit differentiation or proliferation capacity equivalent to young-passage stem cells.

### Examples

Hereinafter, the present invention will be described in more detail with reference to examples. It will be obvious to skilled in the art that these examples are merely to illustrate the present invention, and the scope of the present invention is not limited by these examples.

### Example 1: Characterization of Old Stem Cells

### (1) Change in Cell Morphology

In order to confirm how the cell morphology changes as the senescence of adult stem cells progresses, the cell morphology at each passage was observed under a microscope. Specifically, cells were suspended and cultured in DMEM medium containing 10% FBS and 1% AA/PS, and then washed twice with DPBS. 0.25% trypsin-EDTA was added in an amount corresponding to about 1/6 of the cell culture solution, and the cells were detached by incubation for 3 minutes in an incubator, which was maintained at 36.5°C and injected with 5% CO₂. A neutralizing agent was added in order to inhibit the action of the trypsin-EDTA, and then centrifugation was performed in a centrifuge at a speed of 3,000 rpm and at 4°C for 5 minutes. The collected cells were cultured again in DMEM medium. The culture medium was replaced, and the cells were cultured from passage 6 to passage 23.

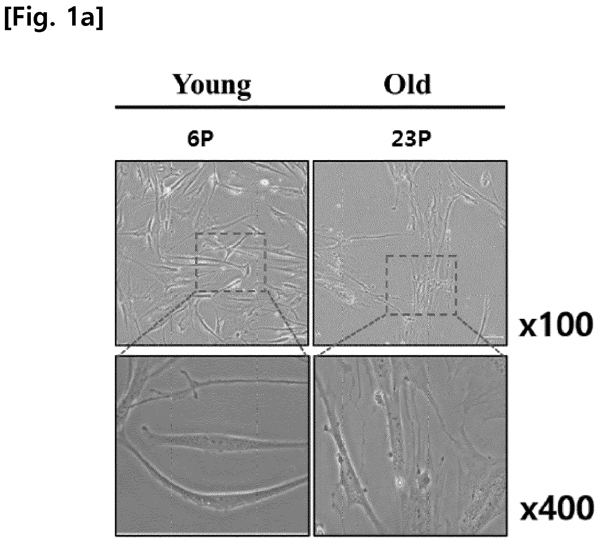

As a result of the culture, it was observed that the adult stem cells had a wide area and a thinner edge as the number of passages increased (FIG. 1a).

### (2) Changes in Expression of Adult Stem Cell Markers

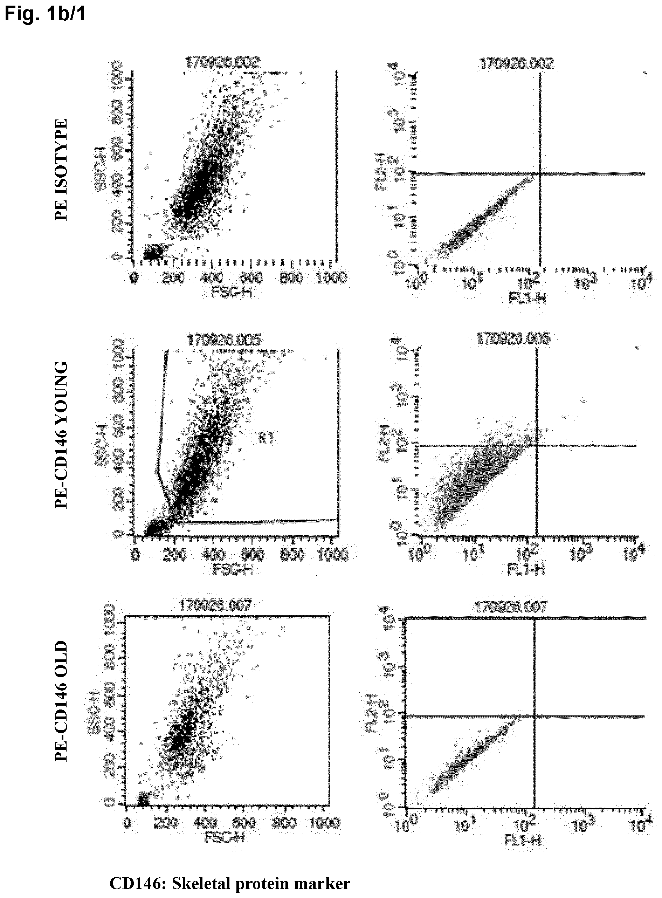

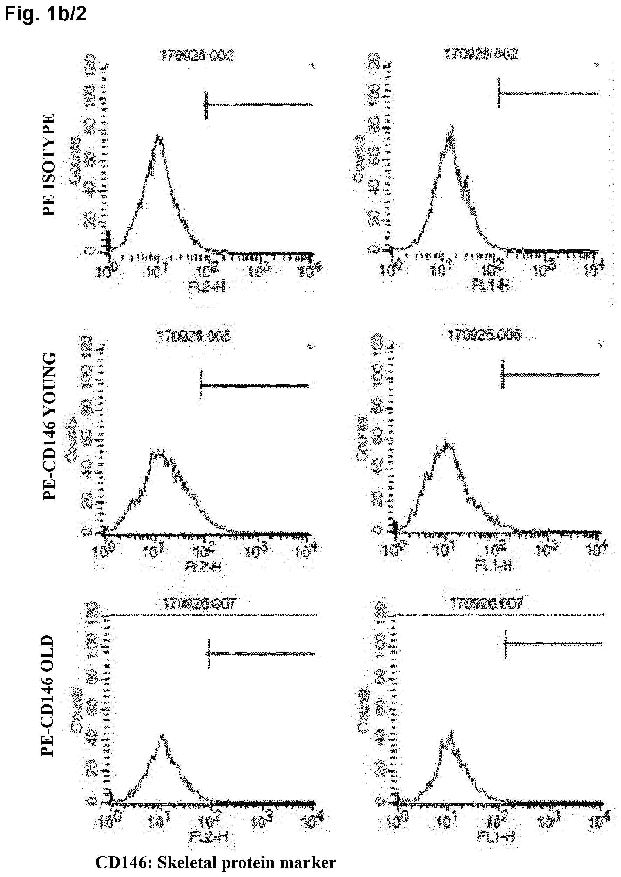

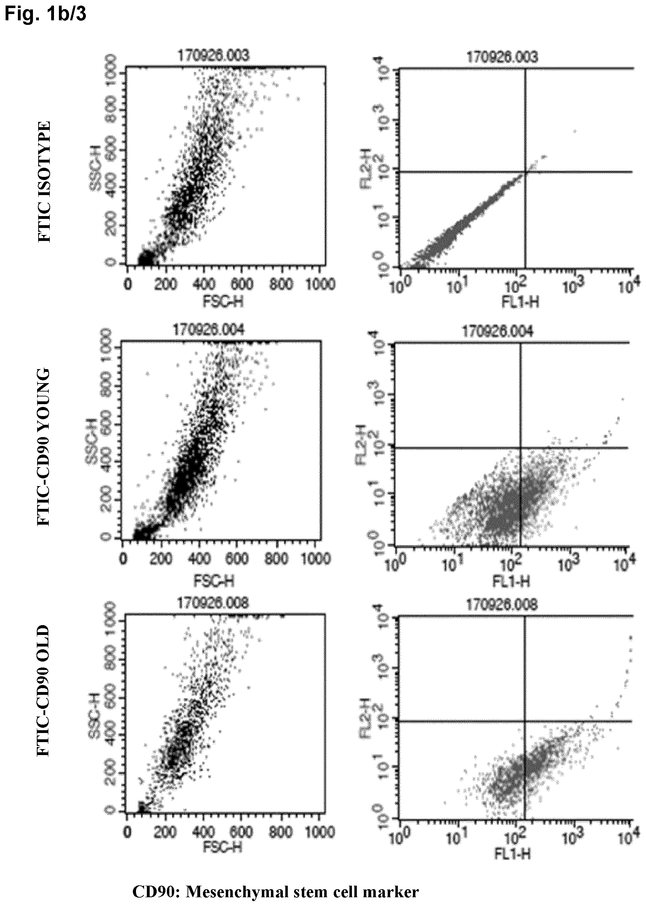

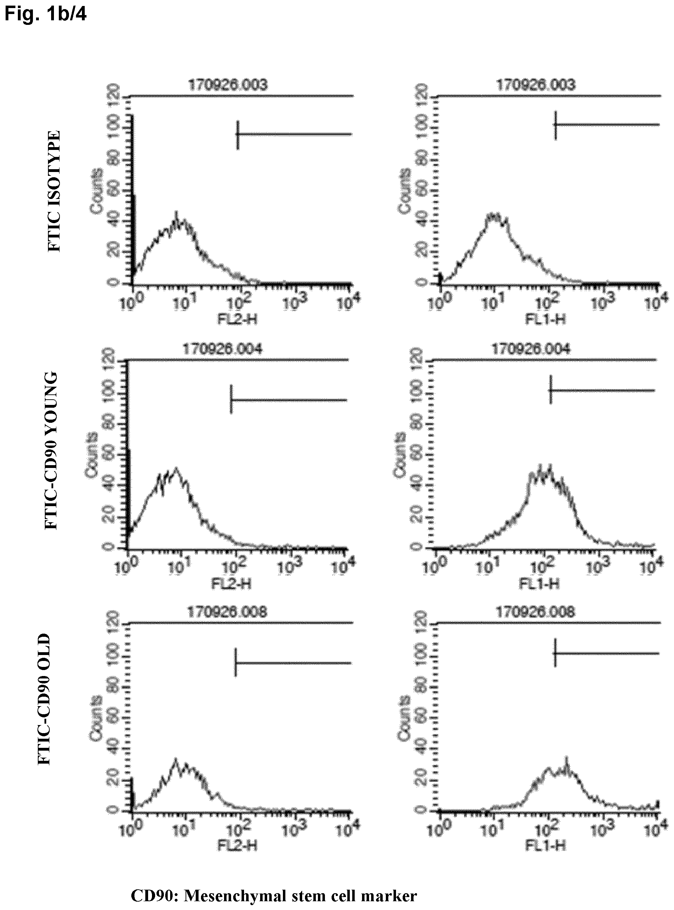

In order to examine changes in the expression of adult stem cell markers, cell surface markers were detected, and flow cytometry shown in FIG. 1b was used. First, adjusted numbers of young cells and old cells were cultured, and the cells subjected to antigen-antibody reactions and fluorescent reactions. CD90 was used as a marker of mesenchymal stem cells, which indicated that differentiation ability decreased as senescence progressed. CD146 was used as a skeletal protein marker, which indicated that the protein constituting the cytoskeleton decreased as senescence progressed.

### (3) Results of Senescence-Associated (SA)-β-Gal Assay

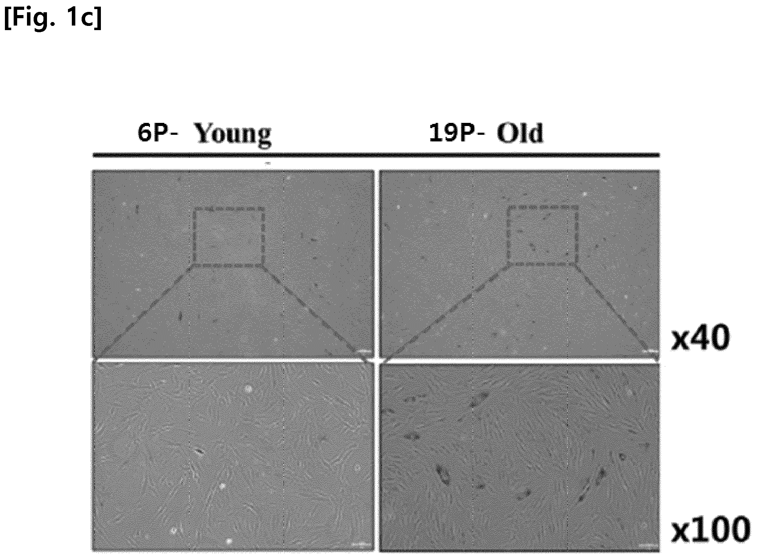

In order to examine whether adult stem cells have the characteristics of old cells during passage, SA-b-gal assay was performed. The same number of cells at each passage were cultured, and the results were derived using X-gal and solution so that old cells would be stained blue (FIG. 1c).

This indicates that, as the number of passages increases, adult stem cells have characteristics of old cells.

### Example 2: Results of Proteomic Analysis

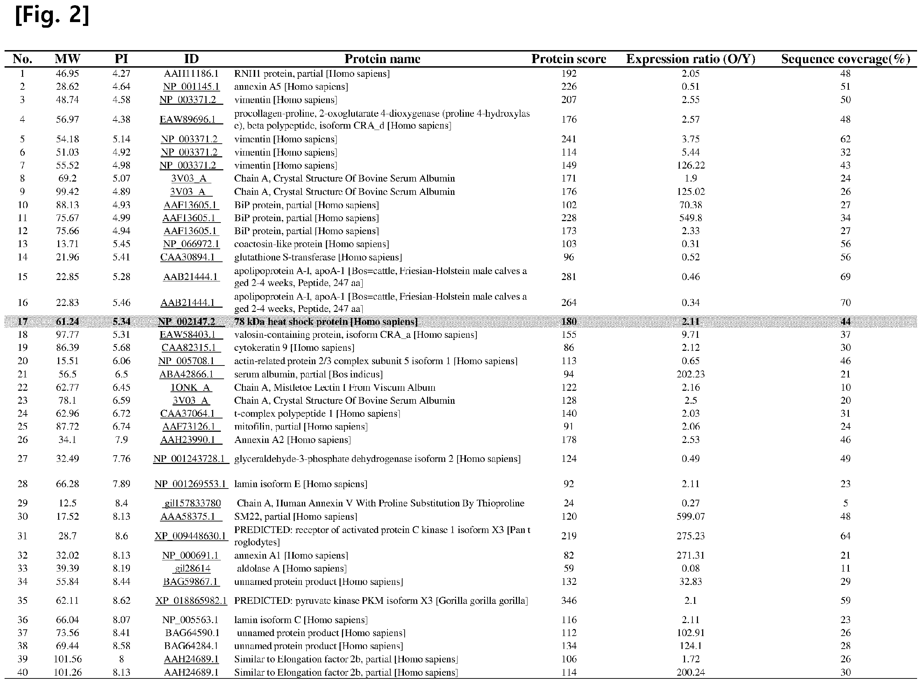

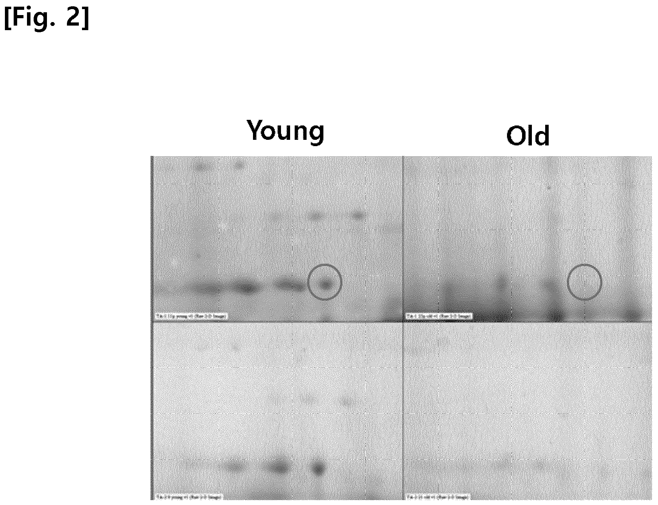

In order to examine the changes in proteomics as the senescence of adult stem cells progresses, proteomic analysis was performed using each cell pellet cultured at each passage. Specifically, the 2DE lysis solution was directly added to quantify the extracted protein, and 100 µg per sample was used, and gels on 2DE were ran and stained with alkaline silver. Image analysis was performed by comparing the stained spots. Quantitative analysis for examining changes in expression of protein spots from the scanned image was performed using PDQuest software (version 7.0, BioRad). The quantity of each spot was normalized by the total valid spot intensity, and protein spots showing at least 2-fold significant changes in expression compared to old cells were selected. As a result of protein identification, glucose-regulated protein 78 (hereinafter referred to as GRP78), which has been shown to be significant, was selected (FIG. 2).

### Example 3: Examination of GRP78 Gene Expression in Old Adult Stem Cells

Reverse transcription polymerase chain reaction (RT-PCR) was used to examine the change in GRP78 expression at the gene level in old adult stem cells. After the adult stem cells at each passage were collected, the cell surface was washed twice with DPBS. Only RNA was extracted using TRIzol, chloroform and isopropanol, and the purity of the RNA extract was increased using 75% EtOH. The resulting RNA extract was synthesized into cDNA, and only the GRP78 gene of the DNA was replicated and amplified by PCR. The amplification product was electrophoresed on a 2% agarose gel containing eco-dye at 100 V for 30 minutes, and the band was visualized with a UV illuminator.

At the same time, the same amount of cDNA was used for each passage, and in order to prove that the entire RT-PCR process was performed consistently, the amount of the extracted cDNA for each passage was used to examine the expression level of the GAPDH gene.

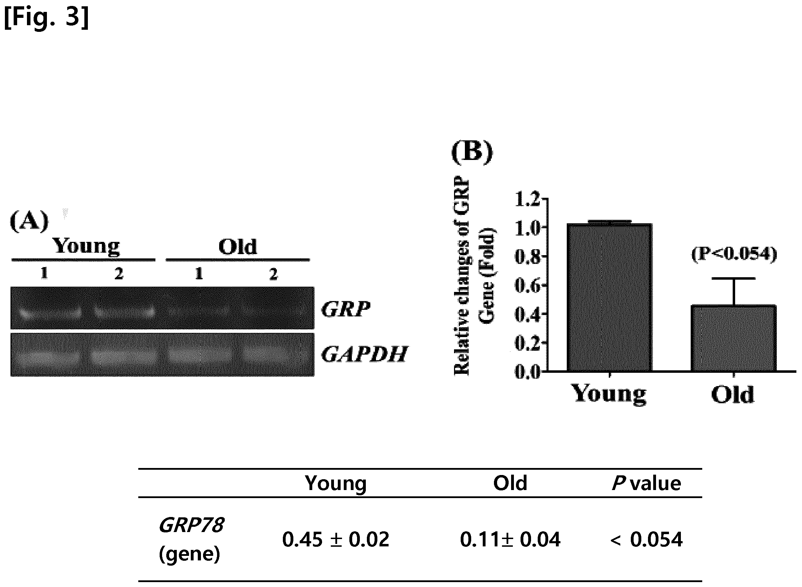

As a result, as shown in FIG. 3, it was confirmed that the genetic level of GRP78 in adult stem cells decreased as senescence progressed.

### Example 4: Examination of GRP78 Protein Expression in Old Adult Stem Cells

Adult stem cells at each passage were collected, and then the cell surface was washed twice with DPBS. Next, the intracellular protein portion was extracted using RIPA buffer (25 mM Tris-HCl Ph 7.6, 150 mM NaCl, 1% NP-40, 1% sodium deoxycholate, 0.1% SDS). The extracted protein was quantified by BCA (bicinchoninic acid) assay using albumin as a standard, and used to examine GRP78 protein expression in the cells.

A 12% SDS (sodium dodecyl sulfate)-polyacrylamide gel (SDS-PAGE) was prepared, and 30 µg of the quantified protein sample for each passage was loaded and electrophoresed on the prepared SDS-PAGE, so that the extracted intracellular proteins were separated according to size. After electrophoresis, the proteins separated according to size on the gel were transferred onto a nitrocellulose membrane by electroblotting.

Among the proteins having different sizes, transferred onto the membrane, a membrane portion corresponding to the size of GRP78 was taken. The taken membrane was put into a blocking buffer obtained by adding 5% skim buffer to TTBS containing a GRP78-specific antibody, and was incubated with the antibody using a shaker at a low temperature of 4°C. After incubation with the primary antibody (diluted at a ratio of 1:1,000), the membrane was additionally incubated with a rabbit-IgG-HRP (horse radish peroxidase)-conjugated secondary antibody (diluted at a ratio of 1:3,000) specific for the primary antibody. Detection was performed by ECL (enhanced chemiluminescence).

At the same time, the same amount of the protein was used for each passage, and in order to prove that the entire Western blotting process was performed consistently, the expression level of the extracted protein for each passage was examined using β-actin antibody.

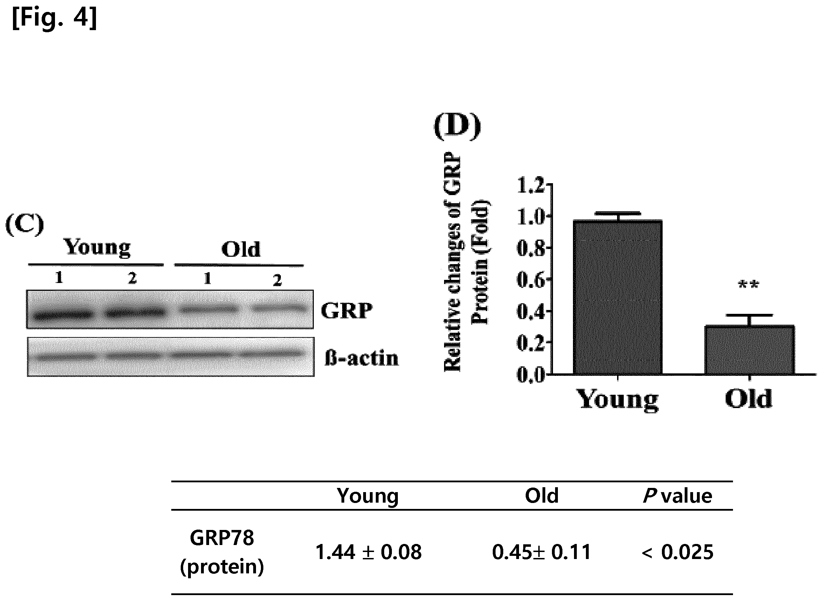

As a result, as shown in FIG. 4, it was confirmed that the level of the GRP78 protein in the adult stem cells decreased as senescence progressed.

### Example 5: Change in Intracellular Distribution of GRP78 by Senescence

Using immunofluorescent assay (IFA), the intracellular distribution of the GRP protein depending on adult stem cell senescence was examined.

Cells at each passage were cultured up to a certain cell number in different wells in 6-well plates covered with a cover glass. The respective plates incubated up to the same cell number were observed under a microscope, and then the culture medium was removed. The cells were washed twice with DPBS to remove the remaining cell debris, and then fixed with 2 ml of 4% paraformaldehyde (PFA) at room temperature for 10 to 20 minutes. The fixed cells were permeabilized by incubation with 0.05 to 0.25% Triton X-100/PBS solution for 10 to 15 minutes. After completion of incubation, the cells were washed three times with PBS for 10 minutes each time, and blocked with 3% BSA (Bovine Serum Albumin) solution for 1 hour. After completion of the blocking, the blocking buffer was completely removed, and moisture at the edge was carefully removed with Kimwipes. GRP78 antibody was diluted at a ratio of 1:100 in 3% BSA/PBS solution and incubated with the cells for 24 hours. At this time, the cells were placed in a 4°C chamber shaker so that the reaction occurred well. After completion of incubation with the primary antibody, the cells were washed three times with PBS for 10 minutes. For secondary antibody binding, a fluorescent-conjugated secondary antibody suitable for the host was added to 3% BSA/PBS solution at a ratio of 1:500 and incubated with the cells. At this time, the plate was placed on a chamber shaker at 4°C, and the humidity was increased to make it humid. After completion of incubation with the secondary antibody, the cells were washed three times with PBS for 10 minutes each time, and stained with DAPI for nucleus staining. To prevent auto-fluorescence, 1 mg/ml sodium borohydride (NaBH₄) in PBS as a quenching solution was added and reacted for 5 minutes. After completion of quenching, the cells were washed three times with PBS for 10 minutes each time, and mounted with mounting solution. The samples were completely dried, and then observed under a confocal microscope.

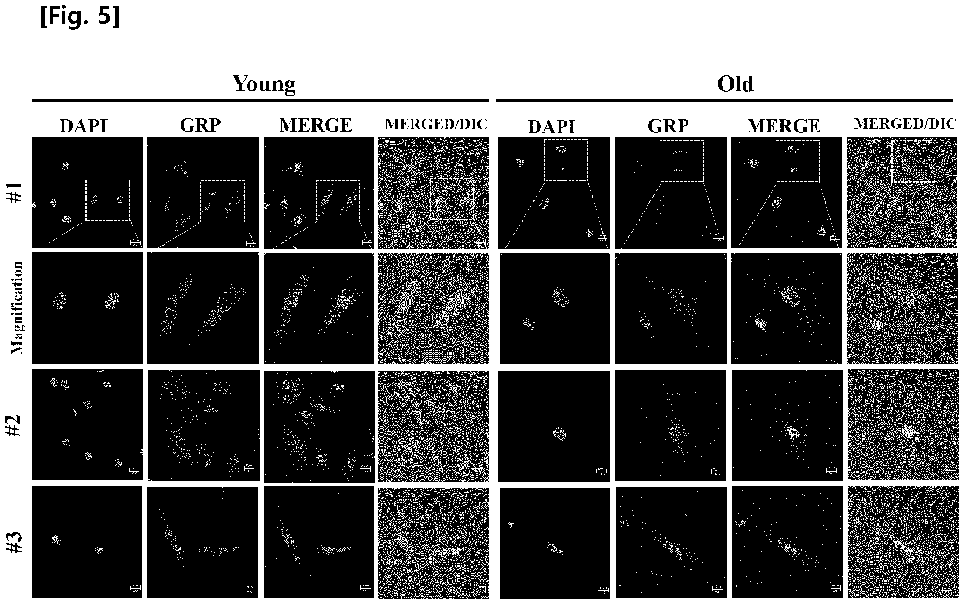

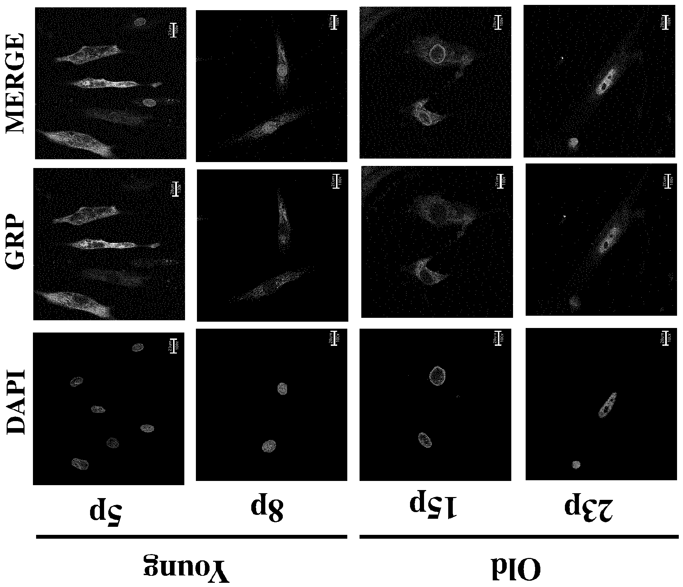

As a result, as can be seen in the images in FIG. 5, it was confirmed that, in the young passage, the GRP78 protein was evenly expressed/distributed in the cytoplasmic region, including the membrane position, and in the older passages, the GRP78 protein was expressed specifically only at the nucleus position. It was confirmed that, as senescence of the cells progressed, translocation of the GRP78 protein in the nucleus to the cytoplasm and membrane decreased.

### Example 6: GRP78 Protein Specific for Adult Stem Cells

According to the method of Example 4 above, adult stem cells, cancer cells and fibroblasts were collected at each passage. The cell surface at each passage was washed twice with DPBS, and then intracellular protein was extracted using RIPA buffer (25 mM Tris-HCl pH 7.6, 150 mM NaCl, 1% NP-40, 1% sodium deoxycholate, 0.1% SDS). The extracted protein was quantified by BCA (bicinchoninic acid) assay using albumin as a standard, and used to examine GRP78 protein expression in the cells.

A 10% SDS (sodium dodecyl sulfate)-polyacrylamide gel (SDS-PAGE) was prepared, and 30 µg of the quantified protein sample for each passage was loaded and electrophoresed on the prepared SDS-PAGE, so that the extracted intracellular proteins were separated according to size. After electrophoresis, the proteins separated according to size on the gel were transferred to a nitrocellulose membrane by electroblotting.

Among the proteins having different sizes, transferred onto the membrane, a membrane portion corresponding to the size of GRP78 was taken. The taken membrane was put into a blocking buffer obtained by adding 5% skim buffer to TTBS containing a GRP78-specific antibody, and was incubated with the antibody using a shaker at a low temperature of 4°C. After incubation with the primary antibody (diluted at a ratio of 1:1,000), the membrane was additionally incubated with a rabbit-IgG-HRP (horse radish peroxidase)-conjugated secondary antibody (diluted at a ratio of 1:3,000) specific for the primary antibody. Detection was performed by ECL (enhanced chemiluminescence).

At the same time, the same amount of the protein was used for each passage, and in order to prove that the entire Western blotting process was performed consistently, the expression level of the extracted protein for each passage was examined using GAPDH and β-actin antibody.

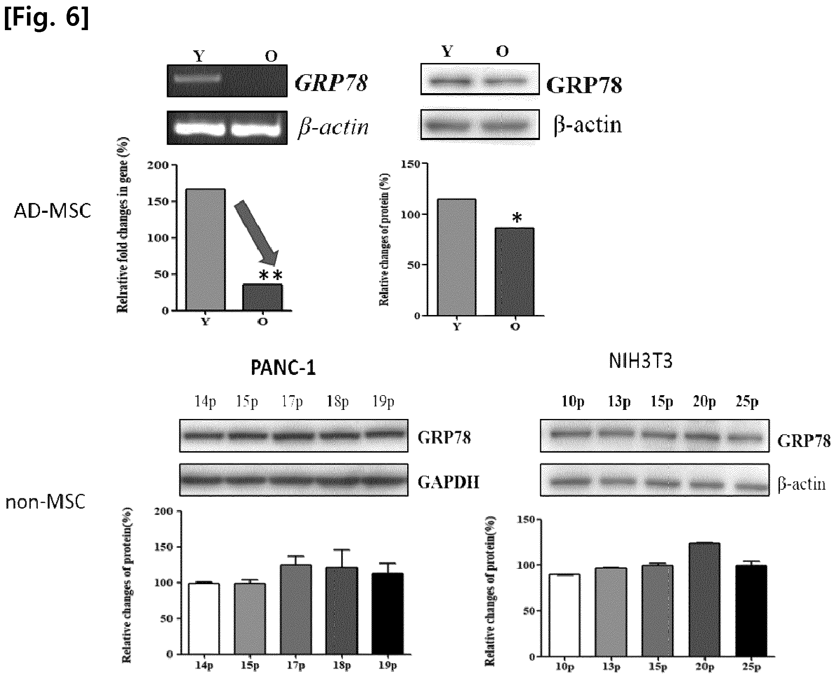

As a result, as shown in FIG. 6, it was confirmed that, as senescence of the adult stem cells progressed, the genetic level of GRP78 in the cells was 0.1, which corresponds to a decrease of about 90% compared to the average value (1) for the young passages, and the average value of the protein levels for the old passages was about 0.7, which corresponds to a decrease of about 30% compared to the average value (1) for the young passages. In the case of the cancer cells and the fibroblasts, it was confirmed that the expression level of the GRP78 protein did not decrease, and increases in the expression level were also not constant, indicating that the expression level did not change.

### Example 7: Expression Level of GRP78 Protein in Each Cell Region

In order to examine the expression level of GRP78 in each cell region in young-passage adult stem cells and old-passage adult stem cells, cytoplasmic and membrane proteins were isolated. All centrifugation procedures were performed at 4°C, and the proteins were stored on ice.

To extract cytoplasmic proteins from old adult stem cells and undifferentiated adult stem cells, 200 µl of cytoplasmic lysis buffer (CST, subcellular fractionation kit) was dispensed, and the cells were scraped off with a scraper. The suspension extracted from the obtained cells was incubated on ice for 20 minutes. The sample was centrifuged using a refrigerated centrifuge at 3,000 rpm for 5 minutes, and the supernatant excluding the pellet was extracted and isolated.

In addition, to extract membrane proteins, 100 µl of cytoplasmic lysis buffer (CST, subcellular fractionation kit) was dispensed to the cell pellet obtained in the previous step, and the pellet was scraped off with a 25 gauge needle. After the pellet was suspended, the sample was centrifuged at 3,000 rpm for 10 minutes. In addition, the supernatant excluding the pellet was extracted and isolated. Using the obtained cytoplasmic proteins and membrane proteins, the Western blot analysis used in Examples 4 and 6 was performed to examine changes in protein expression levels.

As a result, based on the expression level of α-tubulin protein that is used as a standard marker in cytoplasm, it could be confirmed that the expression level of the GRP78 protein in the cytoplasm decreased as senescence progressed. In addition, based on the expression level of ATPase protein that is used as a standard marker in the cell membrane, it could be confirmed that the expression level of the GRP78 protein in the cell membrane decreased as senescence progressed. Through this, it was confirmed that examining the expression level of the GRP78 protein in the cell membrane and isolating adult stem cells expressing a high level of GRP78 in the membrane can be suggested as a method of distinguishing between old stem cells from young stem cells.

### Example 8: GRP78 Isolation by MACS and Examination of Marker Expression

Based on the results confirmed in Example 7 above, in order to selectively isolate the GRP78 protein present in the cell membrane of adult stem cells, adult stem cells expressing the GRP78 protein in the cell membrane were isolated using an MACS (magnetic-activated cell sorting) technique.

In order to isolate GRP78 using a magnet, 1 x 10⁷ adult stem cells were prepared. A primary antibody specific for GRP78 was bound to the prepared adult stem cells, and then the cells were centrifuged using a centrifuge at 300 × g for 10 minutes. The supernatant was removed, and the remaining pellet was suspended in 70 µl of washing buffer (a mixture of MACS washing solution and MACS BSA stock solution). 10 µl of a blocking agent (MACS blocking solution) was added to the suspension to prevent binding to other proteins, and 20 µl of anti-rabbit microbead was added. The resulting mixture was vortexed and incubated in a refrigerator at 2 to 8°C for 15 minutes under a light-shielded condition. After 15 minutes of incubation, 1 ml of washing buffer was added, and the mixture was centrifuged using a centrifuge at 300 × g for 10 minutes. The supernatant was removed, and then the pellet was suspended in 500 µl of washing buffer.

In order to isolate adult stem cells having microbeads attached thereto by the MACS separator, a column was mounted in the magnetic field, and a 15 ml conical tube was mounted below. For washing of the column, 3 ml of washing buffer was added to the column and allowed to descend by gravity, and then a fresh 15 ml conical tube was mounted. The prepared cells were placed in the column and allowed to flow by gravity. At this time, the cells collected in the conical tube were GRP78-negative sorted cells to which the magnetic beads were not attached, and the cells collected in the column were GRP78-positive sorted cells. After the solution in the column descended by gravity, the column was removed from the MACS separator, a fresh 15 ml conical tube was prepared, and a plunger was inserted and pushed into the column to collect GRP78-positive sorted cells.

A fluorescent antibody specific for the GRP78 antibody was attached to the cells isolated by the above method, and then the results were derived using flow cytometry for identification of the marker.

As a result, it was confirmed that GRP78-positive cell group graph and the GRP78-negative cell group graph are separated in the young-passage and old-passage adult stem cells. This can be used to specifically screen only cells in which the GRP78 protein is present in the membrane.

### Industrial Applicability

The use of the protein marker GRP78 according to the present invention makes it possible to isolate only highly efficient stem cells by removing old stem cells. Thus, the protein marker GRP78 may be used as an effective marker for controlling the quality of stem cell therapy products and evaluating the stability and effectiveness thereof, and is useful in the production of stem cell therapy products having excellent therapeutic efficacy.

Although the present invention has been described in detail with reference to specific features, it will be apparent to those skilled in the art that this description is only of a preferred embodiment thereof, and does not limit the scope of the present invention. Thus, the substantial scope of the present invention will be defined by the appended claims and equivalents thereto.

## Claims

1. A method of isolating highly efficient stem cells using an antibody or aptamer specific for GRP78.

2. The method of claim 1, comprising steps of:
(a) binding a GRP78-specific primary antibody to stem cells;
(b) binding a secondary antibody-immobilized magnetic bead to the primary antibody; and
(c) isolating GRP78-expressing stem cells using MACS (magnetic-activated cell sorting).

3. The method of claim 1, wherein the highly efficient stem cells express GRP78 in the membrane.

4. The method of claim 2, wherein step (c) further comprises a step of removing stem cells expressing a low level of GRP78 in the membrane.

5. The method of claim 4, wherein the stem cells expressing a low level of GRP78 in the membrane are old stem cells.

6. A composition for screening highly efficient stem cells, the composition comprising an agent capable of measuring the expression level of mRNA of GRP78 gene or a protein encoded by the gene.

7. The composition of claim 6, wherein the agent capable of measuring the expression level of the mRNA is a primer capable of amplifying the GRP78 gene or a probe that binds specifically to the GRP78 gene.

8. The composition of claim 6, wherein the agent capable of measuring the expression level of the protein is an antibody or aptamer specific for the protein encoded by the GRP78 gene.

9. The composition of claim 6, wherein the highly efficient stem cells are non-senescent young stem cells.

10. A kit for screening highly efficient stem cells, the kit comprising the composition of claim 6.

11. The kit of claim 10, wherein the kit is an RT-PCR kit, a DNA chip kit or a protein chip kit.

12. A method for screening highly efficient stem cells, the method comprising steps of:
(a) extracting a DNA or protein from stem cells;
(b) measuring the expression level of mRNA of GRP78 gene or a protein encoded by the gene;
(c) comparing the measured expression level of mRNA of the gene or the protein encoded by the gene with a control; and
(d) selecting the stem cells as highly efficient stem cells if the expression level increases compared to the control.

13. The method of claim 12, wherein the expression level of mRNA of the gene is measured by any one selected from the group consisting of polymerase chain reaction (PCR), reverse transcription-polymerase chain reaction (RT-PCR), real-time PCR, RNase protection assay (RPA), microarray, and Northern blotting.

14. The method of claim 12, wherein the expression level of the protein is measured by any one selected from the group consisting of Western blotting, radioimmunoassay (RIA), radioimmunodiffusion, enzyme-linked immunosorbent assay (ELISA), immunoprecipitation, flow cytometry, immunofluorescence assay, Ouchterlony assay, complement fixation assay, and protein chip assay.

15. The method of claim 12, wherein the control is old stem cells.

16. The method of claim 12, wherein the highly efficient stem cells are non-senescent young stem cells.
